Europäisches Patentamt

European Patent Office

Office européen des brevets

(19)

(11) Numéro de publication: **0 515 656 B1**

(12) **FASCICULE DE BREVET EUROPEEN**

(45) Date de publication de fascicule du brevet: **14.09.94** (51) Int. Cl.⁵: **A01K 67/033, A01N 63/00**

(21) Numéro de dépôt: **92902331.5**

(22) Date de dépôt: **17.12.91**

(86) Numéro de dépôt internationale :
**PCT/FR91/01024**

(87) Numéro de publication internationale :
**WO 92/10929 (09.07.92 92/17)**

(54) **PROCEDE D'ACTIVATION DE LA MOBILITE DE FEMELLES D'INSECTE OOPHAGE.**

(30) Priorité: **18.12.90 FR 9015841**

(43) Date de publication de la demande:
**02.12.92 Bulletin 92/49**

(45) Mention de la délivrance du brevet:
**14.09.94 Bulletin 94/37**

(84) Etats contractants désignés:
**AT BE CH DE DK ES FR GB GR IT LI LU MC NL SE**

(56) Documents cités:
**FR-A- 2 460 625**

(73) Titulaire: **UNION NATIONALE DES COOPERA-TIVES AGRICOLES D'APPROVISIONNEMENT**
**83-85 avenue de la Grande Armée**
**F-75782 Paris Cédex 16 (FR)**

Titulaire: **INSTITUT NATIONAL DE LA RECHER-CHE AGRONOMIOUE**
**147, rue de l'Université**
**F-75341 Paris Cédex 07 (FR)**

Titulaire: **BASF FRANCE**
**140, rue Jules-Guesde**
**F-92300 Levallois-Perret (FR)**

(72) Inventeur: **FRENOY, Corinne**
**"Le Renouveau" Bât. C3,**
**22, rue de la Gare**
**F-91570 Bièvres (FR)**
Inventeur: **HAWLITZKY, Nicole**
**1, square Palissy**
**F-78330 Fontenay-le-Fleury (FR)**
Inventeur: **LARTAUD, Guy**
**20, rue Michelet**
**F-95120 Ermont (FR)**

(74) Mandataire: **Simonnot, Bernard et al**
**Cabinet Simonnot**
**35 rue de Clichy**
**F-75442 Paris Cédex 09 (FR)**

Rank Xerox (UK) Business Services
(3.10/3.09/3.3.3)

# Description

La présente invention concerne un procédé d'activation de la mobilité des femelles d'un insecte parasitoïde oophage agissant par recherche de son hôte et destruction de ce dernier. Un tel processus est notamment appliqué à la lutte biologique contre la pyrale du maïs Ostrinia nubilalis HÜBNER, par l'intervention de Trichogramma brassicae BEZDENKO (appelé antérieurement maïdis).

## TECHNIQUE ANTERIEURE.

Pour la mise en oeuvre de la lutte biologique ci-dessus, on a été amené à produire en masse, à conserver et à conditionner des oeufs d'un insecte des denrées, tel que Ephestia kuehniella parasité par le trichogramme, en vue de lâchers au champ. L'ensemble de ces dispositions est notamment décrit dans les brevets français n° 79 17 774, 79 17 904, 85 09 251 et 85 12 115. Or dans le cas du trichogramme, les Demandeurs ont trouvé que l'élevage sur hôtee de substitution entraîne une certaine accoutumance du trichogramme à ce dernier et que, de ce fait, le parasite réagit moins à la ponte de la pyrale du maïs lors du lâcher au champ, car il se déplace moins et détecte moins rapidement les pontes de la pyrale. L'efficacité parasitaire par individu au champ en est donc réduite et elle doit être compensée à l'heure actuelle par un accroissement des doses pouvant atteindre un facteur 10, particulièrement coûteux, chaque dose unitaire consistant en une capsule renfermant des oeufs frais d'Ephestia et un inoculum d'oeufs parasités par le trichogramme.

Les Demandeurs ont donc cherché à activer la mobilité de femelles d'insectes entomophages, en particulier du trichogramme, en vue d'aboutir à un procédé permettant d'obvier à l'accoutumance évoquée ci-dessus.

On sait que les kairomones jouent un rôle très important dans la communication inter-spécifique chez les insectes. Dans le cas d'un parasitoïde, elles peuvent intervenir à différentes étapes de la recherche de l'hôte, à savoir, localisation de l'habitat de l'hôte, recherche et reconnaissance de l'hôte.

Des études antérieures ont permis de mettre en évidence un effet d'activation de la mobilité des femelles de Trichogramma brassicae en présence des effluves libérés par la dévagination artificielle de la glande phéromonale de femelles vierges d'Ostrinia nubilalis testées en photophase ou en scotophase lors de l'émission phéromonale (Frenoy et al., 1990). La réponse des parasitoïdes dans cette dernière situation est plus rapide et plus inportante qu'en présence des femelles vierges testées en photophase. Cette mise en évidence a été réalisée en olfactomètre linéaire (Renou et al. 1989) constitué de deux branches indépendantes mises en parallèle afin de comparer l'activité induite par un stimulus chimique à celle induite par de l'air pur en tant que témoin. Ce type d'olfactomètre ne permettant cependant pas de déterminer le rôle précis de ces stimuli chimiques sur le comportement de recherche de l'hôte par les trichogrammes, les Demandeurs ont conçu un autre tue d'olfactomètre qui permet d'étudier ce comportement, traduit par le temps de recherche et la variabilité des réponses en fonction des stimuli chimiques proposés.

Ce type de micro-olfactomètre est constitué d'une enceinte où passe un air purifié balayant une source odorante ou agissant directement en tant que témoin. En amont du flux d'air est déposée une ooplaque d'Ostrinia nubilalis afin de donner une cible biologique à l'insecte, tandis que le comportement des parasitoïdes peut être observé, enregistré et analysé à l'aide d'un moniteur et d'un magnétoscope.

Le parasitoïde a été soumis à différentes sources odorantes telles que le mélange phéromonal libéré par les femelles vierges de pyrales vivantes, pendant les deux dernières heures de la scotophase, élevées en photopériode 16L/8D, ou bien des émanations libérées par la dévagination de la glande phéromonale des femelles vierges testées en photophase (après la huitième heure du début de cette phase), élevées en photopériode 16L/8D.

Ces activités ont été comparées à celles obtenues en présence de deux types de témoins, à savoir, un flux d'air pur balayant l'ooplaque cible, ainsi que les émanations de femelles vierges élevées en photophase continue, ayant l'extrémité abdominale dévaginée, effluves qui n'induisent pas de changement comportemental particulier.

A partir de l'introduction du parasitoïde dans l'enceinte du micro-olfactomètre, on a donc mesuré le temps que met la femelle parasitoïde à retrouver la ponte-hôte et à commencer à la parasiter. Ces tests comportementaux ont été répétés 30 fois et seront explicités plus loin en regard du tableau reporté ci-après.

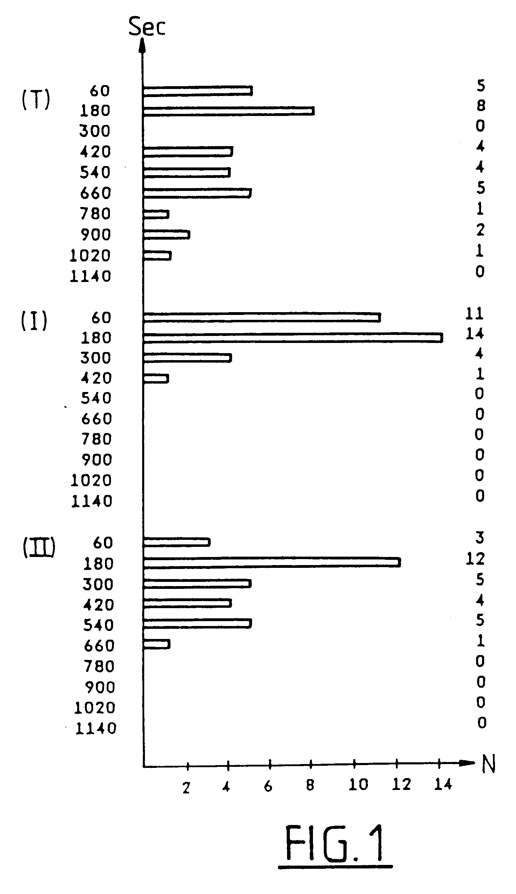

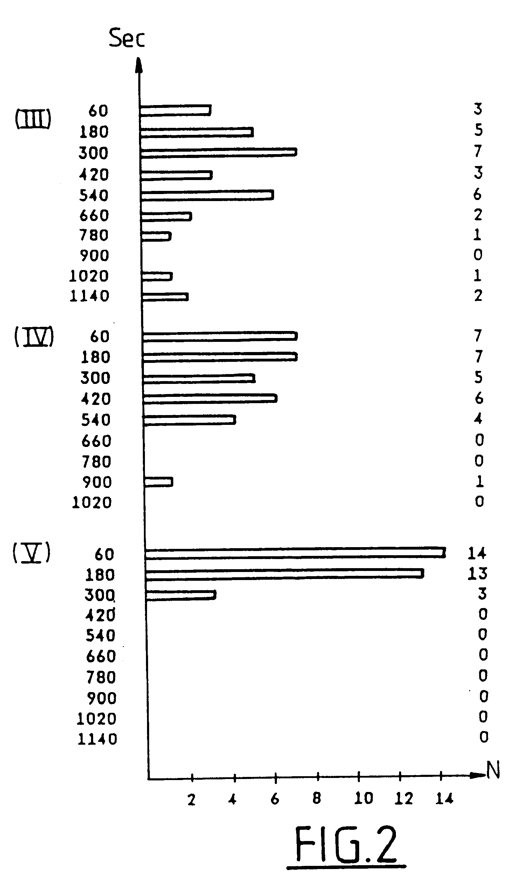

Sur la base de ce mode opératoire, on a tracé un histogramme de fréquences de réponse des trichogrammes aux différentes sources odorantes proposées, corne illustré sur les graphiques des figures 1 et 2 des dessins annexés, relatifs respectivement au témoin (T), aux femelles vierges en scotophase (I), en photophase (II), en photophase continue (III) et aux ooplaques (IV). Sur ces graphiques, on a porté en abscisses le nombre N d'individus par essai, donnant une réponse pour le temps en secondes porté en ordonnées. On peut voir qu'en présence du mélange phéromonal les trichogrammes retrouvent beaucoup plus rapidement l'ooplaque-cible, en comparaison avec le témoin et les effluves émis par la glande dévaginée des femelles élevées en photophase continue.

Par chromatographie et spectrographie de masse, on a alors pu identifier la substance responsable de l'activation de la mobilité et de l'orientation des trichogrammes, comme étant l'acide oléique. Les Demandeurs ont en outre découvert qu'en plus de la mise en présence directe des trichogrammes en développement et des adultes émergés avec l'acide oléique, l'application d'acide oléique au cours de l'élevage de l'hôte Ephestia kuehniella afin de laisser une trace de ce composé dans ou sur les oeufs parasités petit permettre aux trichogrammes produits à partir de tels oeufs de présenter les mêmes caractéristiques que ceux directement soumis audit composé.

EXPOSE DE L'INVENTION.

Conformément à l'invention le procédé d'activation consiste donc à mettre en présence d'acide oléique les femelles de trichogramme issues d'un oeuf-hôte, notamment d'Ephestia kuehniella, par imprégnation préalable dudit oeuf-hôte ou de son environnement immédiat au moyen d'acide oléique.

TABLEAU

| | TEMOIN | | | FEMELLES VIERGES PHOTOPHASE CONTINUE | | | FEMELLES VIERGES EN PHOTOPHASE | | | FEMELLES VIERGES EN APPEL | | | ACIDE OLEIQUE | | |
|---|---|---|---|---|---|---|---|---|---|---|---|---|---|---|---|
| | N | MOYENNE | ECART-TYPE | N | MOYENNE | ECART-TYPE | N | MOYENNE | ECART-TYPE | N | MOYENNE | ECART-TYPE | N | MOYENNE | ECART-TYPE |
| | 50 | 6mn47 | 4mn49 | 50 | 7mn12 | 5mn04 | 30 | 4mn42 | 2mn49 | 30 | 2mn34 | 1mn48 | 30 | 2mn11 | 1mn35 |

Ces résultats montrent très nettement que les molécules libérées par les femelles vierges en période d'émission phéromonale accélèrent le processus de recherche de l'hôte en diminuant de façon importante le temps que met l'insecte à retrouver la ponte-hôte. En effet, il est de 2mn34 en moyenne, temps trois fois plus court que celui du témoin "air pur" qui est de 6 mn47. Par contre, le temps mis par les trichogrammes soumis aux effluves de femelles vierges en photophase (4mn42) est intermédiaire entre celui mis par le témoin et celui induit par les stimuli émanants des femelles vierges en appel. Les effluves de femelles vierges élevées en photophase continue, l'extrémité abdominale dévaginée, n'induisent pas de changement comportemental particulier par rapport au témoin. On remarque que l'acide oléique en tant que source odorante se traduit par une mobilité similaire à celle induite par les femelles vierges en appel. Le profil de réponse en présence d'acide oléique (série V) est similaire à celui obtenu en présence du mélange phéromonal libéré par les femelles vierges en appel (série I).

MANIERE DE REALISER L'INVENTION.

L'imprégnation de l'oeuf-hôte mentionné ci-dessus peut être effectuée au stade de l'oeuf frais stérilisé ou bien après parasitisme par le trichogramme, cette imprégnation pouvant être réalisée au moyen d'une solution d'immersion, d'une pulvérisation ou d'un enrichissement de l'atmosphère d'une enceinte convenable contenant les oeufs.

Dans le cas de l'imprégnation de l'environnement immédiat de l'oeuf-hôte, on peut effectuer une pulvérisation d'acide oléique ou d'une solution convenable sur les supports des oeufs en cours d'élevage ou conditionnés en vue du lâcher au champ.

Il y a lieu de noter que la présence d'acide oléique, pendant le processus d'obtention en masse de l'oeuf-hôte, peut intervenir avantageusement pendant le stade du parasitisme en augmentant la mobilité des parasites, ce qui a pour effet une meilleure répartition et donc un taux de parasitisme supérieur sans superparasitisme. L'effet de mobilité accrue s'assortit en outre d'un effet d'apprentissage, tendant à apprendre aux trichogrammes à reconnaître l'effluve convenable et donc à être plus efficaces dans la recherche des oeufs.

Dans le cas des capsules destinées au lâcher au champ et renfermant un inoculum d'oeufs parasités et des oeufs frais. l'application d'acide oléique peut avoir lieu à différentes périodes, par exemple par pulvérisation de la surface interne ou externe des capsules avant ou après leur paraffinage, pendant le stockage ou pendant l'induction d'arrêt du développement, ou encore par imprégnation de l'aiguille servant à ménager les trous dans les capsules, donc par imbibition des parois des trous dans le carton.

Il est clair que l'application d'acide oléique peut être envisagée en de nombreuses occasions dans la chaîne de production de Trichogramma brassicae, soit pour sa production, soit pour son utilisation au champ.

Les effets bénéfiques pourraient aussi s'observer sur d'autres espèces de trichogrammes ou d'autres parasitoïdes et, éventuellement, sur des prédateurs nourris avec les oeufs d'Ephestia enrichis en acide oléique.

Il est bien entendu que la présente invention n'a été décrite qu'à titre explicatif mais nullement limitatif et qu'on pourra y apporter toute modification utile notamment dans le domaine des équivalences techniques, sans sortir de son cadre.

**Revendications**

1. Procédé d'activation de la mobilité de femelles d'un insecte parasitoïde oophage agissant par recherche de son hôte et destruction de ce dernier, en particulier la recherche de la pyrale du maïs Ostrinia nubilalis par Trichogramma brassicae, issu d'un oeuf-hôte, caractérisé par le fait qu'on met en présence d'acide oléique les femelles de trichogramme issues de l'oeuf-hôte, par imprégnation préalable dudit oeuf-hôte ou de son environnement immédiat.

2. Procédé selon la revendication 1, caractérisé par le fait qu'on effectue l'imprégnation au stade de l'oeuf-hôte, avantageusement après parasitisme par le trichogramme.

3. Procédé selon l'une des revendications 1 ou 2, caractérisé par le fait qu'on effectue l'imprégnation au moyen d'une solution pour immersion, d'une pulvérisation ou d'un enrichissement de l'atmosphère d'une enceinte renfermant les oeufs.

4. Procédé selon la revendication 3, caractérisé par le fait qu'on effectue l'imprégnation par pulvérisation sur les supports des oeufs en cours d'élevage ou conditionnés en capsules de lâcher au champ.

**Claims**

1. A method for activating the mobility of females of an oophageous parasitoid insect acting by seeking and destroying its host, in particular the seeking of the corn borer Ostrinia nubilalis by Trichogramma brassicae, from a host egg, characterised by the fact that the Trichogramma females from the host egg are exposed to oleic acid by first impregnating said host egg or its immediate environment.

2. A method according to Claim 1, characterised by the fact that the impregnation is carried out in the stage of the host egg, advantageously after parasitism by the Trichogramma.

3. A method according to one of Claims 1 or 2, characterised by the fact that the impregnation is carried out by means of a solution for immersion, by spraying or by enriching the atmosphere of an enclosure surrounding the eggs.

4. A method according to Claim 3, characterised by the fact that the impregnation is carried out by spraying on the supports of the eggs during breeding or when packaged in capsules for releasing in the field.

**Patentansprüche**

1. Verfahren zur Aktivierung der Mobilität von Weibchen eines oophagen parasitoiden Insekts, das durch Suche seines Wirts und Vernichtung des letzteren wirkt, insbesondere die Suche von Trichogramma brassicae, die aus einem Wirtsei kommt, nach der Mais-pyralis Ostrinia nubilalis, **dadurch gekennzeichnet**, daß man die aus dem Wirtsei entstandenen Weibchen von Trichogramma durch vorangehende Imprägnierung des Wirtseis oder seiner unmittelbaren Umgebung in Gegenwart von Ölsäure bringt.

2. Verfahren nach Anspruch 1, **dadurch gekennzeichnet**, daß man die Imprägnierung im Stadium des Wirtseis vorteilhafterweise nach Parasitierung durch das Trichogramma durchführt.

3. Verfahren nach einem der Ansprüche 1 oder 2, **dadurch gekennzeichnet**, daß man die Imprägnierung mittels einer Lösung durch Eintauchen, einer Zerstäubung oder durch Anreicherung der Atmosphäre einer die Eier einschließenden Kammer durchführt.

4. Verfahren nach Anspruch 3, **dadurch gekennzeichnet**, daß man die Imprägnierung durch Bestäuben der Träger der Eier im Verlauf der Aufzucht oder der zur Freisetzung im Feld in Kapseln konditionierten Eier durchführt.

FIG.1

FIG.2